# EUROPÄISCHE PATENTSCHRIFT

(11) **EP 1 314 724 B1**
(45) Veröffentlichungstag und Bekanntmachung des Hinweises auf die Patenterteilung: **05.09.2007**
(21) Anmeldenummer: 02022955.5
(22) Anmeldetag: 11.10.2002
(51) Int. Cl.: C07C 317/46, A01N 41/10

(54) **Neue thermodynamisch stabile Kristallmodifikation von (2-(2-Chloro-4-mesyl-benzoyl)cyclohexane-1,3-dione)**
Novel thermodynamically stable crystal modification of (2-(2-Chloro-4-mesyl-benzoyl)cyclohexane-1,3-dione)
Nouvelle modification crystalline thermodynamiquement stable de (2-(2-Chloro-4-mesyl-benzoyl)cyclohexane-1,3-dione)

(30) Priorität: 24.10.2001 DE 10152459
(43) Veröffentlichungstag der Anmeldung: 28.05.2003
(73) Patentinhaber: Bayer CropScience AG, 40789 Monheim (DE)
(72) Erfinder: Eble, Axel, Dr., 50668 Köln (DE); Seidel, Erika, Dr., 53639 Königswinter (DE); Olenik, Britta, Dr., 46242 Bottrop (DE); Benet-Buchholz, Jordi, Dr., 51375 Leverkusen (DE); Hinz, Martin-Holger, 42499 Hückeswagen (DE)

(56) Entgegenhaltungen:
- EP-A- 0 137 963
- MITCHELL G ET AL: "MESOTRIONE: A NEW SELECTIVE HERBICIDE FOR USE IN MAIZE" PEST MANAGEMENT SCIENCE, ELSEVIER, BARKING, GB, Bd. 57, Nr. 2, 1. Februar 2001 (2001-02-01), Seiten 120-128, XP001011689 ISSN: 1526-498X
- LIN Y ET AL: "SAR studies of 2-o-substituted-benzoyl- and 2-alkanoyl-cyclohexane-1, 3-diones as inhibitors of 4-hydroxyphenylpyruvate dioxygenase" BIOORGANIC & MEDICINAL CHEMISTRY LETTERS, OXFORD, GB, Bd. 10, Nr. 9, Mai 2000 (2000-05), Seiten 843-845, XP004199027 ISSN: 0960-894X

## Beschreibung

Die Erfindung betrifft die Kristallmodifikation II von [2-(2-chloro-4-mesylbenzoyl)-cyclohexane-1,3-dione] (im Folgenden als Sulcotrione bezeichnet), Verfahren zu ihrer Herstellung und deren Verwendung als Herbizid.

Die Polymorphie von Wirkstoffen ist von großer Bedeutung für die chemische Entwicklung und die Entwicklung von Formulierungen. Es ist bekannt, dass einige organische Verbindungen in nur einer Kristallstruktur, andere (sog. Polymorphe) in zwei oder mehr Strukturen vorkommen können. Es ist nicht möglich, die Zahl der Kristallmodifikationen einschließlich ihrer physikalisch-chemischen Eigenschaften vorherzusagen, besonders ihre thermodynamische Stabilität, wie auch das unterschiedliche Verhalten nach Darreichung in lebenden Organismen.

Es ist bekannt, dass für einige Polymorphe eine bestimmte Modifikation über den gesamten Temperaturbereich bis zum Schmelzpunkt die thermodynamisch stabile Phase darstellt, wohingegen bei anderen Stoffsystemen ein oder mehrere Übergangspunkte existieren, bei dem sich das Stabilitätsverhältnis umkehrt. Es ist nicht möglich, das Stabilitätsverhältnis und insbesondere die Existenz und Lage von oben bezeichneten Übergangspunkten vorherzusagen. Ein aktueller Überblick über den Stand des Wissens zu diesen prinzipiellen thermodynamischen Verhältnissen ist in J. Bernstein, R.J. Davey, J.O. Henck, Angew. Chem. Int. Ed., 1999, 38, 3440-3461 gegeben.

Sulcotrione besitzt herbizide Eigenschaften und eignet sich für die Herstellung von Pflanzenschutzpräparaten, die zur Unkrautbekämpfung herangezogen werden. Bei der Herstellung des Sulcotrione gemäß Beispiel 1 aus EP-A1-0 137 963, wo Sulcotrione das erste Mal beschrieben wird, fällt Sulcotrione in einer metastabilen Modifikation an, die im weiteren als Kristallmodifikation I bezeichnet wird.

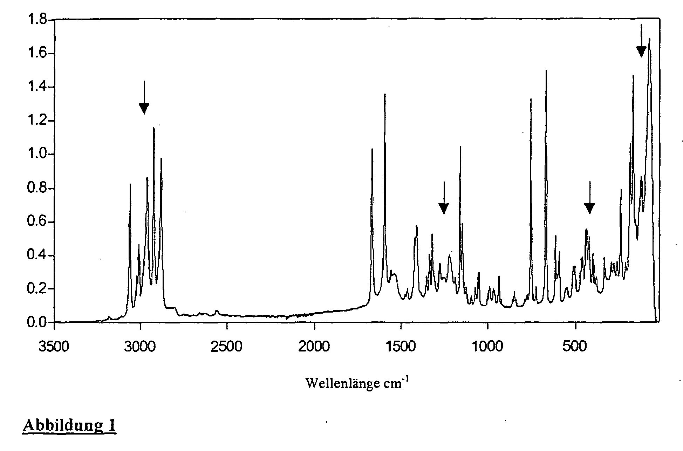

Die Kristallmodifikation I des Sulcotrione hat einen Schmelzpunkt von 144,6°C (DSC, Heizrate 10 K min-1) und ein charakteristisches Raman-Spektrum (Abb. 1). Im Rahmen der vorliegenden Erfindung wurde festgestellt, dass die Kristallmodifikation I des Sulcotrione metastabil ist und deshalb keine geeignete Kristallmodifikation bezüglich Herstellung, Lagerung und Formulierung darstellt.

Metastabile Kristallmodifikationen, wie die Modifikation I des Sulcotrione haben generell Nachteile im Vergleich zu einer thermodynamisch stabilen Form bezüglich des Herstellprozesses, sowie bei der Lagerung und beim Transport der Wirkstoffe und Formulierungen. Aus J. Halebian, W. McCrone, J. Pharm. Sci. 58 (1969) 911 ist bekannt, dass beim Einsatz einer thermodynamisch metastabilen Form bei der Herstellung oder Lagerung eine vollständige oder teilweise Umwandlung in eine andere polymorphe Form stattfinden kann. Als Begleiterscheinung wird dabei unerwünschtes Kristallwachstum (Rekristallisation), Veränderungen in der Bioverfügbarkeit, Verbackungen, usw. beobachtet. Die Umwandlung kann dabei über einen längeren Zeitraum oder spontan erfolgen und kann nicht vorhergesagt werden. Ob, wann, und in welcher Menge eine andere Kristallmodifikation entsteht, bleibt weitgehend dem Zufall überlassen. Dieses Verhalten metastabiler Kristallmodifikationen kann einen großen Einfluss auf die Entwicklung, den Transport und insbesondere auf die Lagerstabilität haben.

In der vorliegenden Erfindung wurde nun eine Modifikation mit einem Schmelzpunkt von 141,4 °C (DSC, Heizrate 10 K min⁻¹) des Sulcotrione gefunden, die über einen breiten Druckbereich und für Temperaturen kleiner 100°C thermodynamisch stabil ist und deshalb besonders geeignet ist für den Einsatz in Formulierungen von Pflanzenschutzpräparaten, wie z.B. Suspensionsformulierungen. Diese neue, in hoher Reinheit erhältliche Modifikation wird im Folgenden als Kristallmodifikation II bezeichnet.

Gegenstand der Erfindung ist daher die Kristallmodifikation II von 2-(2-chloro-4-mesylbenzoyl)cyclohexane-1,3-dione der Formel 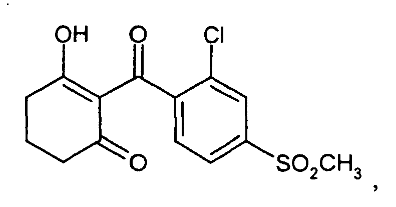 welches im tautomeren Gleichgewicht zur Ketonform mit der Formel 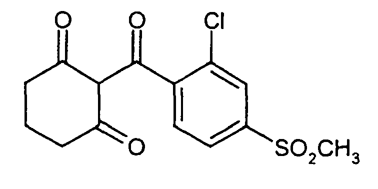 steht, und welches in EP-A1-0 137 963 beschrieben ist. Das Verfahren zur Herstellung des Sulcotrione kann z.B. dem Dokument EP-A2-186 117 entnommen werden.

### Beschreibung der Abbildungen

- Abbildung 1: (Abb. 1) zeigt das Raman-Spektrum der Kristallmodifikation I von Sulcotrione. Die Peakmaxima sind mit Pfeilen gekennzeichnet, die zugehörigen Werte dieser Maxima in Wellenzahlen finden sich in Tabelle 1.
- Abbildung 2: (Abb. 2) zeigt das Raman-Spektrum der Kristallmodifikation II von Sulcotrione. Die Peakmaxima sind mit Pfeilen gekennzeichnet, die zugehörigen Werte dieser Maxima in Wellenzahlen finden sich in Tabelle 1.
- Abbildung 3: (Abb.3) zeigt die aus der Einkristallstrukturanalyse ermittelte Kristallstruktur der Kristallmodifikation II von Sulcotrione. Die wichtigsten Parameter, die die Kristallstruktur beschreiben, finden sich in Tabelle 2.
- Abbildung 4: (Abb.4) zeigt die aus der Einkristallstrukturanalyse ermittelte Kristallpackung der Kristallmodifikation II von Sulcotrione. Die wichtigsten Parameter, die die Kristallpackung beschreiben, finden sich in Tabelle 3.

### Beschreibung der Erfindung

Die Schmelzpunkte wurden mittels DSC (Pyris 1 der Fa. Perkin Elmer, Heizrate 10 K min⁻¹) ermittelt. Zur Bestimmung der Raman-Spektren wurden mittels eines RFS 100/S FT-Raman der Fa. Bruker von jeder Partie mindestens zwei Spektren mit jeweils 128 Scans aufgenommen. Die Feststoffdichte wurde nach der Dichtebestimmungsmethode SOP 5024 mit dem Ultrapyknometer 1000 T der Fa. Quanta-Chrome bestimmt bzw. aus der Einkristallröntgenstrukturanalyse (EKS) ermittelt. Die Einkristallröntgenstrukturanalyse (EKS) wurde durch Verwendung einer Drehanode M18X-HF mit MoKα-Strahlung von MACScience Co und einen SMART-CCD-1000-Detektor von Bruker-AXS bestimmt. Die Daten wurden mit den Programmen SAINT-NT V 5.0 (Datenreduktion, Bruker-AXS) und SADABS (Absorptionskorrektur, Bruker-AXS) bearbeitet. Die Strukturlösung und Verfeinerung wurde mit SHELXTL NT-Version V5.1 durchgeführt.

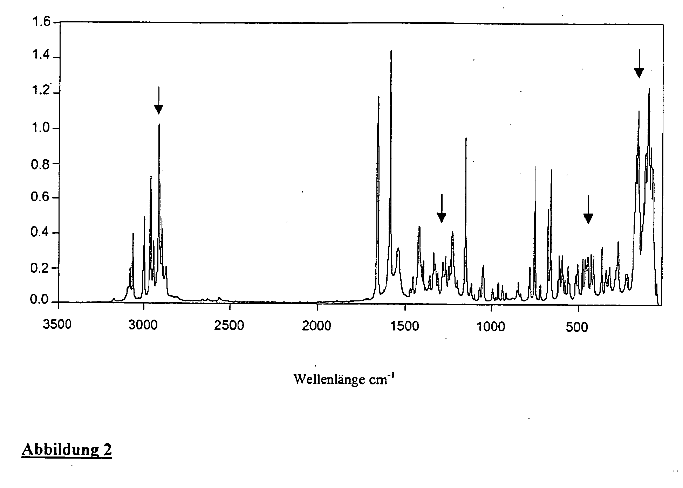

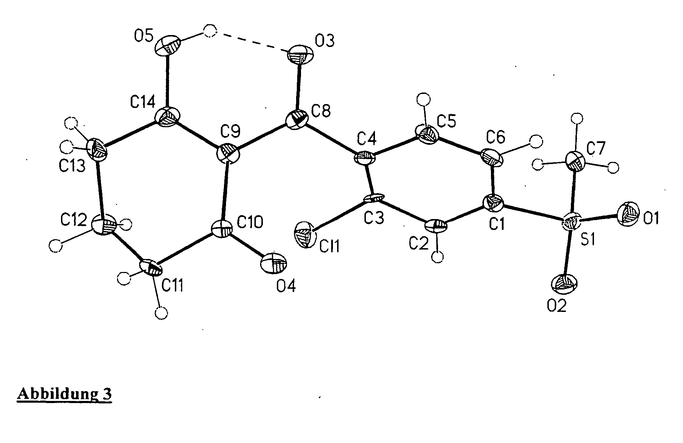

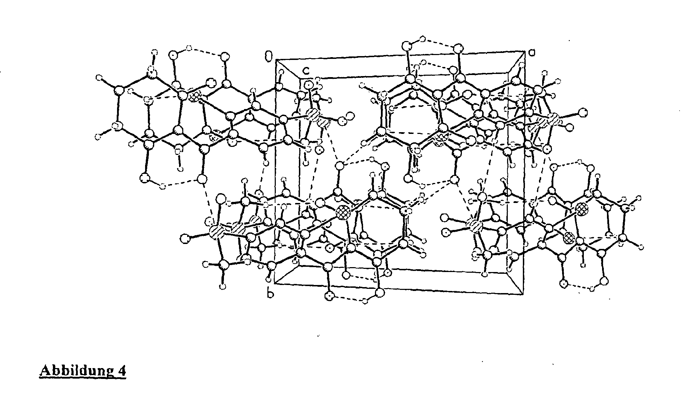

Die im Folgenden gezeigten Tabellen 1 bis 4 stellen die für die Kristallmodifikation II kennzeichnenden Meßwerte zusammen. Sie sind den Werten der Kristallmodifikation I gegenübergestellt, um die Unterschiede zu verdeutlichen. Die Herkunft der Werte der Tabelle 1 sind in den Abbildungen 1 und 2 veranschaulicht. Tabelle 2 gibt die Kenndaten der in Abbildung 3 gezeigten Kristallstruktur wieder. Tabelle 3 bezieht sich auf die Kristallpackung wie in Abb. 4 gezeigt.

**Tabelle 1: Banden der Raman-Spektren in [cm⁻¹]**

| **Modifikation II** | | | **Modifikation I** | |
|---|---|---|---|---|
| 3175 | 992 | | 3062 | 960 |
| 3096 | 958 | | 3010 | 937 |
| 3083 | 935 | | 2963 | 921 |
| 3067 | 845 | | 2927 | 859 |
| 3002 | 779 | | 2884 | 849 |
| 2965 | 752 | | 1671 | 778 |
| 2948 | 718 | | 1594 | 757 |
| 2918 | 676 | | 1559 | 726 |
| 2900 | 660 | | 1541 | 671 |
| 2874 | 613 | | 1461 | 616 |
| 1660 | 594 | | 1419 | 594 |
| 1589 | 578 | | 1410 | 552 |
| 1543 | 560 | | 1352 | 517 |
| 1458 | 513 | | 1337 | 506 |
| 1422 | 503 | | 1321 | 468 |
| 1397 | 476 | | 1279 | 460 |
| 1358 | 460 | | 1251 | 441 |
| 1337 | 447 | | 1223 | 426 |
| 1322 | 429 | | 1189 | 403 |
| 1310 | 415 | | 1160 | 386 |
| 1283 | 371 | | 1147 | 339 |
| 1266 | 346 | | 1125 | 300 |
| 1247 | 328 | | 1094 | 285 |
| 1228 | 280 | | 1071 | 266 |
| 1152 | 236 | | 1050 | 244 |
| 1117 | 227 | | 991 | 217 |
| 1050 | | | 967 | |

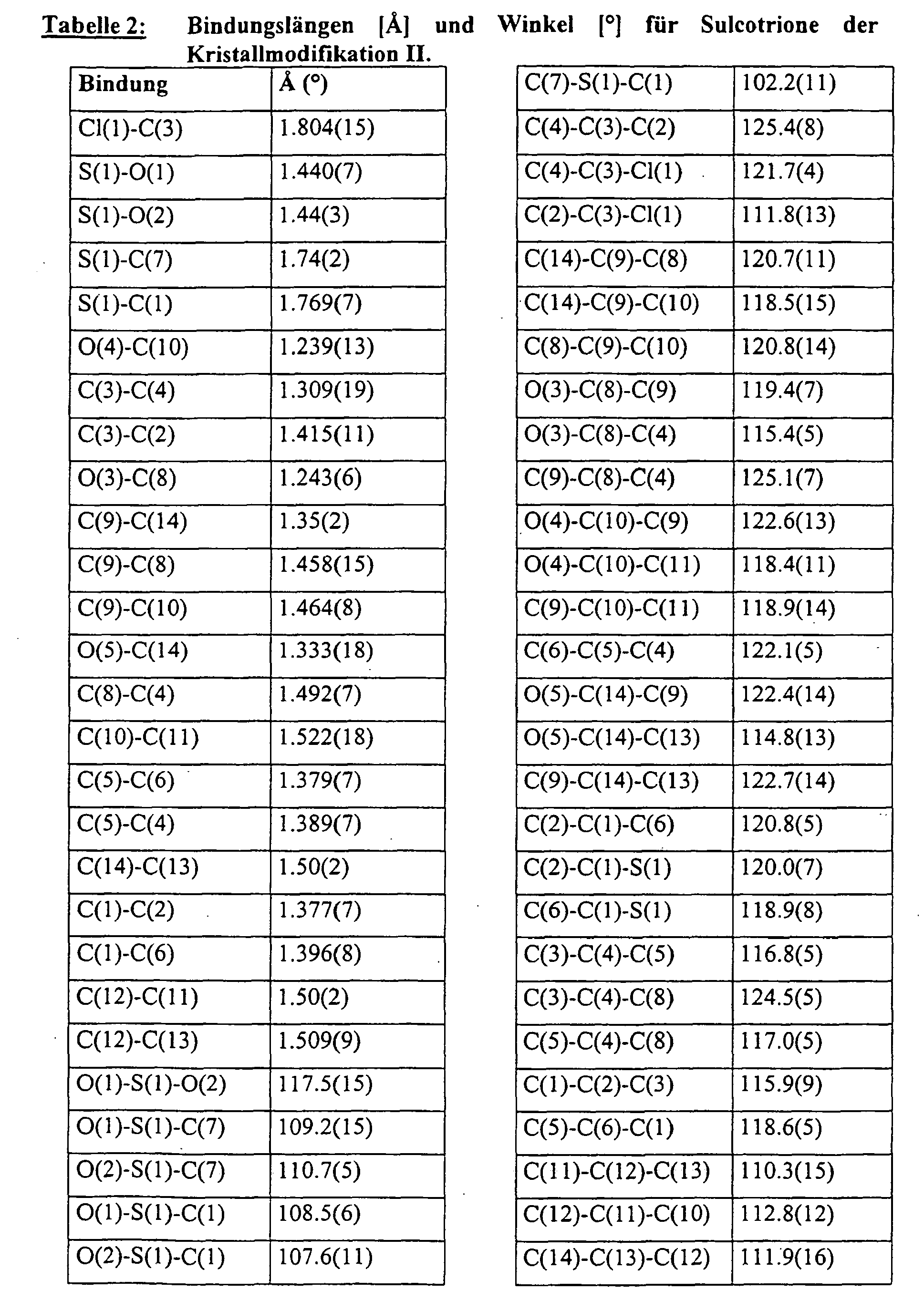

**Tabelle 3: Kristallographische Daten der Kristallmodifikation II von Sulcotrione (Kristallpackung)**

| **Symmetrietyp** | Monoklin | |
|---|---|---|
| **Raumgruppe** | *P*2₁/c | |
| **Dimensionen der Elementarzelle** | a = 11.6415(14) Å | α = 90°. |
| | b = 9.8054(9) Å | β = 115.196(5)°. |
| | c = 13.1078(16) Å | γ = 90° |
| **Volumen der Elementarzelle** | 1353.9(3) Å³ | |
| **Koordinationszahl Z** | 4 | |
| **Dichte (rechnerisch)** | 1.613 Mg/m³ | |

**Tabelle 4: Feststoffdichte der Kristallmodifikationen I und II**

| **Polymorph** | **Dichte** |
|---|---|
| (Modifikation II), experimentell | 1.536 g/ml |
| (Modifikation II), berechnet aus EKS | 1.608 g/ml |
| | |
| (Modifikation I), experimentell | 1.434 g/ml |
| (Modifikation I), berechnet aus EKS | 1.510 g/ml |

Wie die Abbildungen 1 und 2 sowie die Tabellen 1 bis 4 zeigen, hat die Kristallmodifikation II im Vergleich zur Kristallmodifikation I über den klar unterscheidbaren Schmelzpunkt hinaus auch ein klar unterscheidbares Raman-Spektrum und eine klar unterscheidbare Feststoffdichte.

Die thermodynamische Stabilität der Kristallmodifikation II bei Temperaturen kleiner 100°C gegenüber der Kristallmodifikation I lässt sich sowohl aus dem Verhältnis der Feststoffdichten und dem Verhältnis der Schmelzpunkte ermitteln, als auch experimentell überprüfen. Bei Vorlage einer wässrigen Suspension der Kristallmodifikation I, die z.B. über einen Zeitraum von 24 Stunden bei 90°C und 1,013 bar gerührt wird, zeigt sich, dass innerhalb dieses Zeitraums eine quantitative Umwandlung zur Modifikation II stattgefunden hat. Hingegen zeigt sich bei Vorlage einer wässrigen Suspension der Kristallmodifikation II, die über den gleichen Zeitraum von 24 Stunden bei 1,013 bar gerührt wird, dass sowohl bei 90°C, als auch bei 100°C keine Umwandlung erfolgt. Gleichartige Versuche bei Vorlage einer wässrigen Suspension der Kristallmodifikation II, über einen Zeitraum von 24 Stunden bei 110°C bzw. bei 120°C führen zu einer chemischen Zersetzung des Wirkstoffs. Die Kristallmodifikation II stellt somit bei Temperaturen kleiner 100°C die stabile Kristallmodifikation dar.

Gegenstand der Erfindung ist weiterhin ein Verfahren zur Herstellung der Kristallmodifikation II von Sulcotrione, wobei man die Kristallmodifikation I von Sulcotrione in Wasser und/oder inerten organischen Lösungsmitteln suspendiert und/oder löst und bis zur quantitativen Umwandlung in die Modifikation II bei Temperaturen von 0°C bis 80°C behandelt.

Inerte organische Lösungsmittel, die in diesem Verfahren verwendet werden können, sind z.B. niedere Alkohole wie z.B. Methanol, Ethanol, 2-Propanol oder Ketone wie z.B. 2-Butanon, die auch in Mischung mit Wasser verwendet werden können. Als niedere Alkohole werden hier solche Verbindungen bezeichnet, die eins bis zehn Kohlenstoffatome aufweisen, bevorzugt eins bis fünf Kohlenstoffatome. Als niedere Ketone werden hier solche Verbindungen bezeichnet, die drei bis zehn Kohlenstoffatome aufweisen, bevorzugt drei bis sechs Kohlenstoffatome.

Die Umwandlung in die Kristallmodifikation II erfolgt bei Temperaturen kleiner 100°C, bevorzugt bei Temperaturen von 0°C bis 80°C, ganz besonders bevorzugt bei Temperaturen von 60°C bis 80°C. Die Dauer der Umwandlung hängt ab von der Temperatur und der Art des Lösungsmittels. Weiterhin hängt die Dauer der Umwandlung davon ab, ob Impfkristalle der Kristallmodifikation II verwendet werden.

Im allgemeinen kann die Umwandlung zur Modifikation II bei vollständiger Auflösung der Kristalle der Modifikation I bei erhöhter Temperatur durch Kühlungskristallisation zur Raumtemperatur ohne die Verwendung von Impfkristallen direkt erzielt werden. Das Abkühlen auf Raumtemperatur erfolgt vorzugsweise mit einer Kühlrate von kleiner 25°C pro Stunde, besonders bevorzugt mit einer Kühlrate von kleiner 15°C pro Stunde. Die Umwandlung einer Suspension von Kristallen der Modifikation I kann in der Regel ohne die Verwendung von Impfkristallen in einem Zeitraum von 14 Tagen herbeigeführt werden. Werden bei der Umwandlung einer Suspension Impfkristalle der Modifikation II verwendet, ist im Allgemeinen eine Behandlungsdauer von 24 bis 48 Stunden ausreichend, um eine quantitative Umwandlung der Kristalle in die Kristallmodifikation II zu erreichen. Es ist natürlich möglich, die Behandlungsdauer zu verlängern.

Die erhaltenen Kristalle der Kristallmodifikation II werden schließlich abgetrennt und zur Entfernung des Lösungsmittels bei Raumtemperatur oder erhöhter Temperatur bis zur Gewichtskonstanz getrocknet.

Die Kristallmodifikation II eignet sich aufgrund ihrer Stabilität hervorragend für die Zubereitung von Formulierungen als Pflanzenschutzpräparat. Gegenstand der Erfindung sind daher auch Pflanzenschutzpräparate, welche die Kristallmodifikation II von Sulcotrione alleine oder in Mischung mit Hilfs- und Trägerstoffen, sowie in Mischung mit anderen Wirkstoffen enthalten. Die Erfindung schließt auch Mischungen der Kristallmodifikation II des Sulcotrione mit der Kristallmodifikation I des Sulcotrione ein, z.B. solche, die an irgendeiner Stelle des erfindungsgemäßen Umwandlungsverfahrens der Kristallmodifikation I in die Modifikation II auftreten. Aus Stabilitätsgründen sollte die Kristallmodifikation II jedoch keine größeren Anteile der Modifikation I enthalten. Bevorzugt wird eine Wirkstoffqualität mit weniger als 20 Gew.-% der Kristallmodifikation I des Sulcotrione, besonders bevorzugt mit weniger als 10 Gew.-%, ganz besonders bevorzugt mit weniger als 5 Gew.-% und am meisten bevorzugt mit weniger als 2 Gew.-% bei der Formulierung eingesetzt.

Gegebenenfalls wird Sulcotrione in der Kristallmodifikation II mit einem oder mehreren anderen Herbiziden gemischt. Auch solche Mischungen profitieren von den vorteilhaften Eigenschaften der Kristallmodifikation II.

Aufgrund ihrer Stabilität eignet sich die Kristallmodifikation II des Sulcotrione ganz allgemein als Ausgangsmaterial für die Herstellung jedweder Sulcotrione enthaltender Pflanzenschutzformulierungen, auch wenn das Sulcotrione nach der Formulierung nicht mehr in dieser Form, sondern etwa in gelöster Form vorliegt.

Gegenstand der Erfindung sind daher ferner auch Verfahren zur Herstellung von Sulcotrione enthaltenden Pflanzenschutzformulierungen, welche die Kristallmodifikation II von Sulcotrione verwenden sowie Sulcotrione enthaltende Pflanzenschutzformulierungen, die aus der Kristallmodifikation II des Sulcotrione erhalten wurden. Durch den Einsatz der Kristallmodifikation II wird die Sicherheit für Zubereitungen des Sulcotrione erhöht und somit das Risiko falscher Dosierungen verringert.

Die Kristallmodifikation II des Sulcotrione kann in bekannter Weise in die üblichen Formulierungen überführt werden, wie Suspensionskonzentrate, Kolloidale Konzentrate, Dispergierbare Konzentrate, Emulgierbare Konzentrate (Emulsionskonzentrate), Emulsionsbeizen, Suspensionsbeizen, Granulate, Mikrogranulate, Suspoemulsionen, Wasserlösliche Granulate, Wasserlösliche Konzentrate und Wasserdispergierbare Granulate, unter Verwendung geeigneter Hilfs- und Trägerstoffe oder Lösemittel. Hierbei soll die wirksame Verbindung in einer Konzentration von etwa 0,5 bis 90 Gew.-% der Gesamtmischung vorhanden sein, d.h. in Mengen, die ausreichend sind, um den notwendigen Dosierungsspiegel zu erreichen. Die Formulierungen werden beispielsweise hergestellt durch Verstrecken der Kristallmodifikation II des Sulcotrione mit Lösemitteln und/oder Trägerstoffen, gegebenenfalls unter Verwendung von Emulgier- und/oder Dispergiermitteln, und/oder anderen Hilfsstoffen, wie z.B. Penetrationshilfsmitteln.

Die Anwendung erfolgt in der üblichen Weise, indem die unerwünschten Pflanzen und/oder ihr Lebensraum mit dem Wirkstoff bzw. dessen Formulierung in Kontakt gebracht werden.

Sulcotrione in der Kristallmodifikation II zeigt eine hervorragende herbizide Wirkung gegenüber Vertretern der Gruppe sowohl der monokotylen als auch der dikotylen Unkräuter. Gegenstand der Erfindung ist daher auch die Verwendung der Kristallmodifikation II des Sulcotrione zur Herstellung eines Pflanzenschutzpräparates zur Behandlung des Unkrautbefalls.

Die erfindungsgemäße Kristallmodifikation II des Sulcotrione oder Mischungen derselben mit der Kristallmodifikation I oder anderen herbiziden Verbindungen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, insbesondere jedoch Zea.

Die Verwendung der erfindungsgemäßen Kristallmodifikation II des Sulcotrione ist jedoch keineswegs auf diese Gattungen beschränkt, sondern erstreckt sich in gleicher Weise auch auf andere Pflanzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit der erfindungsgemäßen Kristallmodifikation II des Sulcotrione erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen oder Aufstreichen.

Die erfindungsgemäße Kristallmodifikation II des Sulcotrione kann, wie bereits oben ausgeführt, in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent des Wirkstoffs in der erfindungsgemäßen Modifikation II, vorzugsweise zwischen 0,5 und 90 %.

Die erfindungsgemäße Kristallmodifikation II des Sulcotrione kann als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfüron, Beflubutamid, Benazolin (-ethyl), Benfüresate, Bensulf uron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die erfindungsgemäße Kristallmodifikation II des Sulcotrione kann als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäße Kristallmodifikation II des Sulcotrione kann sowohl vor als auch nach dem Auflaufen der Pflanzen appliziert werden. Sie kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen - , besseres Pflanzenwachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Soja, Kartoffel, Baumwolle, Raps sowie insbesondere Mais sowie Obstpflanzen (mit den Früchten Äpfel, Birnen, Zitrusfrüchten und Weintrauben) erwähnt, wobei insbesondere Mais, aber auch Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus *Bacillus thuringiensis* (z.B. durch die Gene CryIA(a), CryIA(b), CryIA(c), CryIIA, CryIIIA, CryIIIB2, Cry9c, Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien v.a. Maissorten, jedoch ebenso Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien v.a. Maissorten, jedoch ebenso Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die erfindungsgemäße Herstellung der Kristallmodifikation II des Sulcotrione geht aus den nachfolgenden Beispielen hervor.

### Beispiele

### Beispiel 1

5 g Sulcotrione der Kristallmodifikation I, welche bei der Herstellung gemäß EP-A2-0 186 117 erhalten wird, werden in 100 g Methanol suspendiert. Die Suspension wird auf 60°C temperiert bis sich die Kristalle der Kristallmodifikation I vollständig gelöst haben. Durch anschließendes Abkühlen auf Raumtemperatur mit einer Kühlrate kleiner 20°C/h kristallisiert der Wirkstoff Sulcotrione in der Kristallmodifikation II. Das Kristallisat wird abfiltriert und bei Temperaturen kleiner 60°C getrocknet. Zur Prüfung auf quantitative Umwandlung wird ein Raman-Spektrum aufgenommen (vgl. Abb. 1 und 2).

### Beispiel 2

5 g Sulcotrione der Kristallmodifikation I werden in 100 g Ethanol suspendiert. Die Suspension wird auf 75°C temperiert bis sich die Kristalle der Kristallmodifikation I vollständig gelöst haben. Durch anschließendes Abkühlen auf Raumtemperatur mit einer Kühlrate kleiner 20°C/h kristallisiert der Wirkstoff Sulcotrione in der Kristallmodifikation II. Das Kristallisat wird abfiltriert und bei Temperaturen kleiner 60°C getrocknet. Zur Prüfung auf quantitative Umwandlung wird ein Raman-Spektrum aufgenommen (vgl. Abb. 1 und 2).

### Beispiel 3

3 g Sulcotrione der Kristallmodifikation I werden in 100 g 2-Propanol suspendiert. Die Suspension wird auf 75°C temperiert bis sich die Kristalle der Kristallmodifikation I vollständig gelöst haben. Durch anschließendes Abkühlen auf Raumtemperatur mit einer Kühlrate kleiner 20°C/h kristallisiert der Wirkstoff Sulcotrione in der Kristallmodifikation II. Das Kristallisat wird abfiltriert und bei Temperaturen kleiner 60°C getrocknet. Zur Prüfung auf quantitative Umwandlung wird ein Raman-Spektrum aufgenommen.

### Beispiel 4

30 g Sulcotrione der Kristallmodifikation I werden in 100 g 2-Butanon suspendiert. Die Suspension wird auf 75°C temperiert bis sich die Kristalle der Kristallmodifikation I vollständig gelöst haben. Durch anschließendes Abkühlen auf Raumtemperatur mit einer Kühlrate kleiner 20°C/h kristallisiert der Wirkstoff Sulcotrione in der Kristallmodifikation II. Das Kristallisat wird abfiltriert und bei Temperaturen kleiner 60°C getrocknet. Zur Prüfung auf quantitative Umwandlung wird ein Raman-Spektrum aufgenommen.

### Beispiel 5

5 g Sulcotrione der Kristallmodifikation I werden in 100 g destilliertem Wasser suspendiert. Die Suspension wird für 2 Wochen auf 80°C temperiert. Das Kristallisat wird abfiltriert und bei Temperaturen kleiner 60°C getrocknet. Zur Prüfung auf quantitative Umwandlung wird ein Raman-Spektrum aufgenommen.

Die vorstehend genannten Beispiele geben verschiedene Möglichkeiten zur Herstellung der Kristallmodifikation II des Sulcotrione wieder. Die Verfahren sind jedoch nicht auf die dort angegebenen Zeiten, Temperaturen, Lösungsmittel etc. beschränkt.

## Patentansprüche

1. Thermodynamisch stabile Kristallmodifikation von 2-(2-Chloro-4-mesylbenzoyl)cyclohexane-1,3-dione (Sulcotrione) der Formel (I) 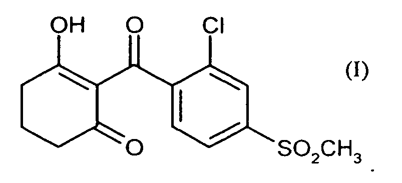 mit einen Raman-Spektrum mit folgenden Peakmaxima in [cm⁻¹]:
| | |
|---|---|
| 3175 | 992 |
| 3096 | 958 |
| 3083 | 935 |
| 3067 | 845 |
| 3002 | 779 |
| 2965 | 752 |
| 2948 | 718 |
| 2918 | 676 |
| 2900 | 660 |
| 2874 | 613 |
| 1660 | 594 |
| 1589 | 578 |
| 1543 | 560 |
| 1458 | 513 |
| 1422 | 503 |
| 1397 | 476 |
| 1358 | 460 |
| 1337 | 447 |
| 1322 | 429 |
| 1310 | 415 |
| 1283 | 371 |
| 1266 | 346 |
| 1247 | 328 |
| 1228 | 280 |
| 1152 | 236 |
| 1117 | 227 |
| 1050 | |

2. Thermodynamisch stabile Kristallmodifikation von Sulcotrione gemäß Anspruch 1 mit einem Schmelzpunkt von 141,4°C (DSC, Heizrate 10 K min⁻¹).

3. Thermodynamisch stabile Kristallmodifikation von Sulcotrione gemäß Anspruch 1 oder 2, **dadurch gekennzeichnet, dass** die Elementarzelle folgende Dimensionen aufweist:
| | |
|---|---|
| a = 11.6415(14) Å | α = 90°. |
| b = 9.8054(9) Å | β = 115.196(5)°. |
| c = 13.1078(16) Å | γ = 90° |

4. Thermodynamisch stabile Kristallmodifikation von Sulcotrione gemäß einem der Ansprüche 1 bis 3 mit folgenden Bindungslängen [Å] und -winkeln [°]:

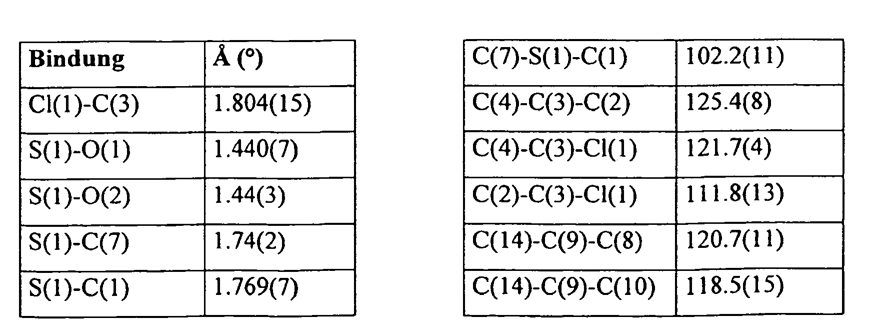

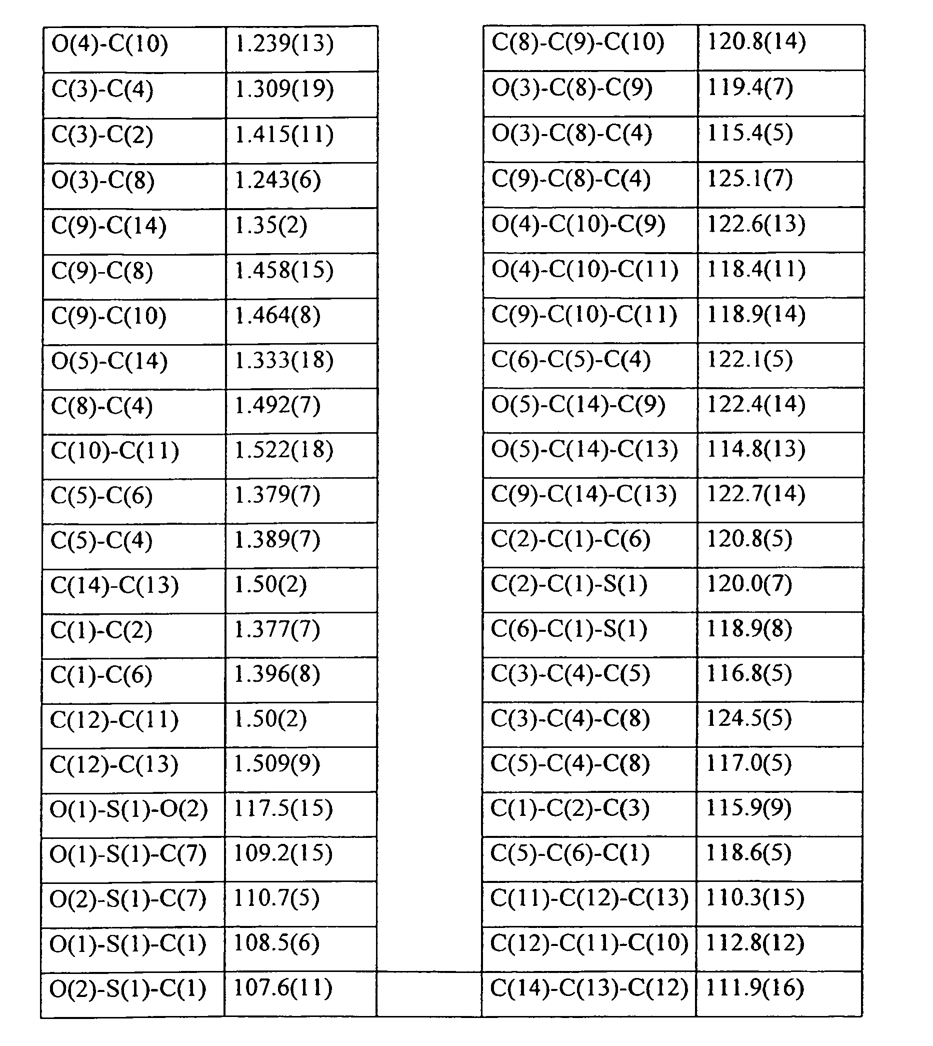

5. Thermodynamisch stabile Kristallmodifikation von Sulcotrione gemäß einem der Ansprüche 1 bis 4 mit einer Feststoffdichte von 1.536 g/ml.

6. Verfahren zur Herstellung der thermodynamisch stabilen Kristallmodifikation von Sulcotrione gemäß Anspruch 1, **dadurch gekennzeichnet, dass** man
a) die metastabile Kristallmodifikation I von Sulcotrione in Wasser und/oder inerten organischen Lösungsmitteln suspendiert und/oder löst und
b) bis zur quantitativen Umwandlung in die thermodynamisch stabile Kristallmodifikation bei Temperaturen von 0°C bis 80°C behandelt.

7. Verfahren gemäß Anspruch 6, **dadurch gekennzeichnet, dass** als Lösungsmittel Alkohole mit 1 bis 10 Kohlenstoffatomen oder Ketone mit 3 bis 10 Kohlenstoffatomen verwendet werden.

8. Herbizides Mittel, **gekennzeichnet durch** einen Gehalt an thermodynamisch stabiler Kristallmodifikation von Sulcotrione gemäß einem der Ansprüche 1 bis 5 und gängigen Streckmitteln und/oder oberflächenaktiven Hilfsstoffen.

9. Herbizides Mittel, umfassend die thermodynamisch stabile Kristallmodifikation von Sulcotrione gemäß einem der Ansprüche 1 bis 5 und die metastabile Kristallmodifikation von Sulcotrione, **dadurch gekennzeichnet, dass** das Gemisch weniger als 20 Gew.-% der metastabilen Kristallmodifikation enthält.

10. Verwendung der thermodynamisch stabilen Kristallmodifikation von Sulcotrione gemäß einem der Ansprüche 1 bis 5 oder eines Mittels gemäß Anspruch 8 oder 9 zur Bekämpfung von unerwünschten Pflanzen.

11. Verfahren zur Bekämpfung von unerwünschten Pflanzen, **dadurch gekennzeichnet, dass** man die thermodynamisch stabile Kristallmodifikation des Sulcotrione gemäß einem der Ansprüche 1 bis 5 oder ein Mittel gemäß Anspruch 8 oder 9 auf die unerwünschten Pflanzen und/oder ihren Lebensraum einwirken lässt.

## Claims

1. A thermodynamically stable crystal modification of 2-(2-chloro-4-mesylbenzoyl)cyclohexane-1,3-dione (sulcotrione) of the formula (I) 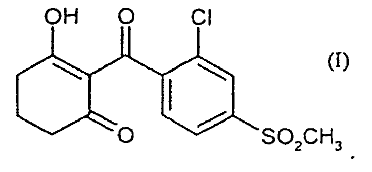 having a Raman spectrum with the following peak maxima in [cm⁻¹]:
| | |
|---|---|
| 3175 | 992 |
| 3096 | 958 |
| 3083 | 935 |
| 3067 | 845 |
| 3002 | 779 |
| 2965 | 752 |
| 2948 | 718 |
| 2918 | 676 |
| 2900 | 660 |
| 2874 | 613 |
| 1660 | 594 |
| 1589 | 578 |
| 1543 | 560 |
| 1458 | 513 |
| 1422 | 503 |
| 1397 | 476 |
| 1358 | 460 |
| 1337 | 447 |
| 1322 | 429 |
| 1310 | 415 |
| 1283 | 371 |
| 1266 | 346 |
| 1247 | 328 |
| 1228 | 280 |
| 1152 | 236 |
| 1117 | 227 |
| 1050 | |

2. A thermodynamically stable crystal modification of sulcotrione as claimed in claim 1 having a melting point of 141.4°C (DSC, heating rate 10 K min⁻¹).

3. A thermodynamically stable crystal modification of sulcotrione as claimed in claim 1 or 2, wherein the unit cell has the following dimensions:
| | |
|---|---|
| a = 11.6415(14) Å | α = 90° |
| b = 9.8054(9) Å | β = 115.196(5)° |
| c = 13.1078(16) Å | γ = 90° |

4. A thermodynamically stable crystal modification of sulcotrione as claimed in any of claims 1 to 3 having the following bond lengths (A) and bond angles [°]:

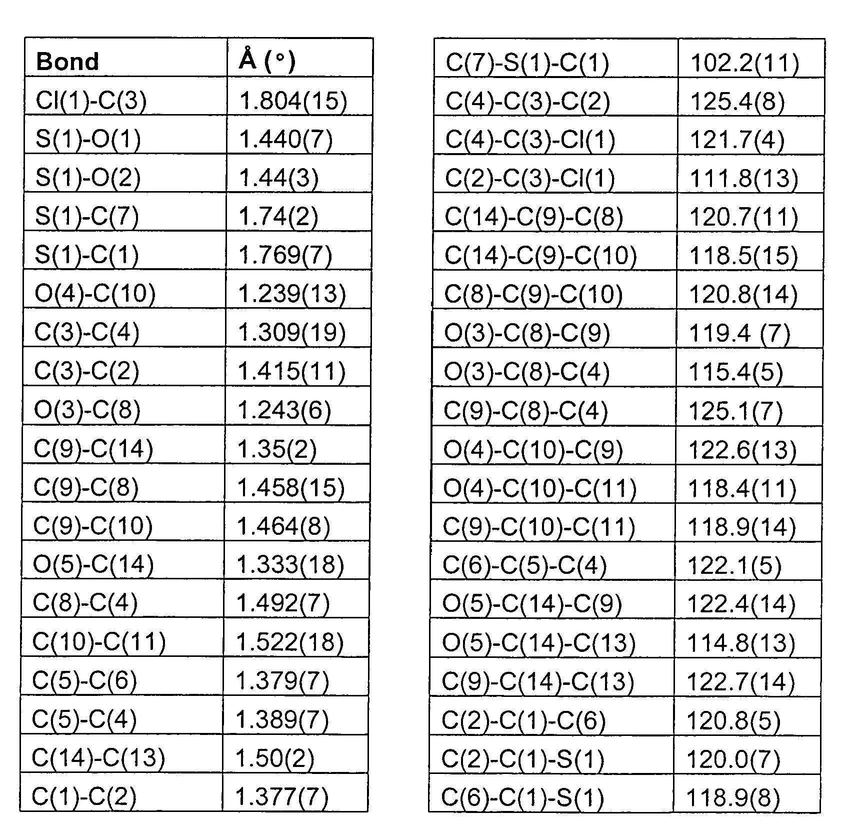

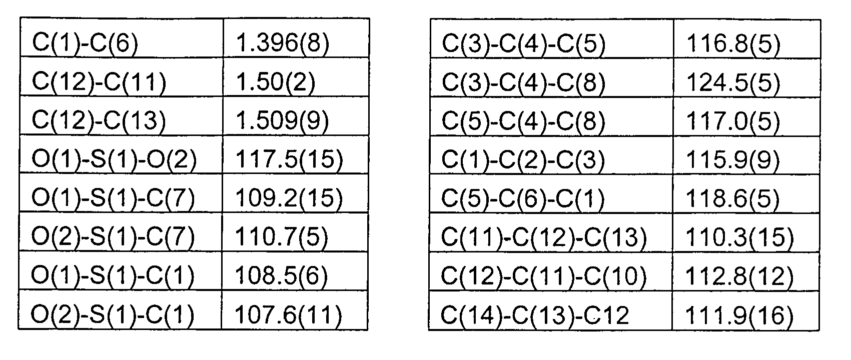

5. A thermodynamically stable crystal modification of sulcotrione as claimed in any of claims 1 to 4 having a density of 1.536 g/ml.

6. A process for the preparation of the thermodynamically stable crystal modification of sulcotrione as claimed in claim 1, wherein
a) the metastable crystal modification I of sulcotrione is suspended and/or dissolved in water and/or inert organic solvents and
b) treated at temperatures of from 0°C to 80°C up to quantitative conversion into the thermodynamically stable crystal modification.

7. A process as claimed in claim 6, wherein alcohols having 1 to 10 carbon atoms or ketones having 3 to 10 carbon atoms are used as solvents.

8. A herbicidal composition comprising a content of thermodynamically stable crystal modification of sulcotrione as claimed in any of claims 1 to 5 and customary extenders and/or surface-active auxiliaries.

9. A herbicidal composition comprising the thermodynamically stable crystal modification of sulcotrione as claimed in any of claims 1 to 5 and the metastable crystal modification of sulcotrione, wherein the mixture contains less than 20% by weight of the metastable crystal modification.

10. The use of the thermodynamically stable crystal modification of sulcotrione as claimed in any of claims 1 to 5 or of a composition as claimed in claim 8 or 9 for controlling undesired plants.

11. A method for controlling undesired plants, wherein the thermodynamically stable crystal modification of sulcotrione as claimed in any of claims 1 to 5 or a composition as claimed in claim 8 or 9 is allowed to act on the undesired plants and/or their habitat.

## Revendications

1. Modification cristalline thermodynamiquement stable de 2-(2-chloro-4-mésylbenzoyl)cyclohexane-1,3-dione (Sulcotrione) de formule (I) 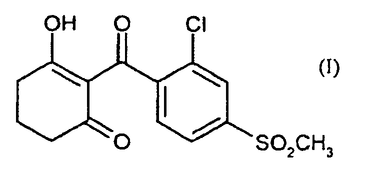 ayant un spectre Raman avec les maximums suivants de pics en [cm⁻¹] :
| | |
|---|---|
| 3175 | 992 |
| 3096 | 958 |
| 3083 | 935 |
| 3067 | 845 |
| 3002 | 779 |
| 2965 | 752 |
| 2948 | 718 |
| 2918 | 676 |
| 2900 | 660 |
| 2874 | 613 |
| 1660 | 594 |
| 1589 | 578 |
| 1543 | 560 |
| 1458 | 513 |
| 1422 | 503 |
| 1397 | 476 |
| 1358 | 460 |
| 1337 | 447 |
| 1322 | 429 |
| 1310 | 415 |
| 1283 | 371 |
| 1266 | 346 |
| 1247 | 328 |
| 1228 | 280 |
| 1152 | 236 |
| 1117 | 227 |
| 1050 | |

2. Modification cristalline thermodynamiquement stable de Sulcotrione selon la revendication 1 avec un point de fusion de 141,4 °C (DSC, vitesse de chauffe 10 K min⁻¹).

3. Modification cristalline thermodynamiquement stable de Sulcotrione selon la revendication 1 ou 2, **caractérisée en ce que** la cellule élémentaire présente les dimensions suivantes :
| | |
|---|---|
| a = 11,6415(14 )Å | α = 90°. |
| b = 9,8054(9) Å | β = 115,196(5)° |
| c = 13,1078(16)Å | γ = 90° |

4. Modification cristalline thermodynamiquement stable de Sulcotrione selon l'une des revendications 1 à 3, avec les longueurs [Å] et angles [°] de liaison suivants .

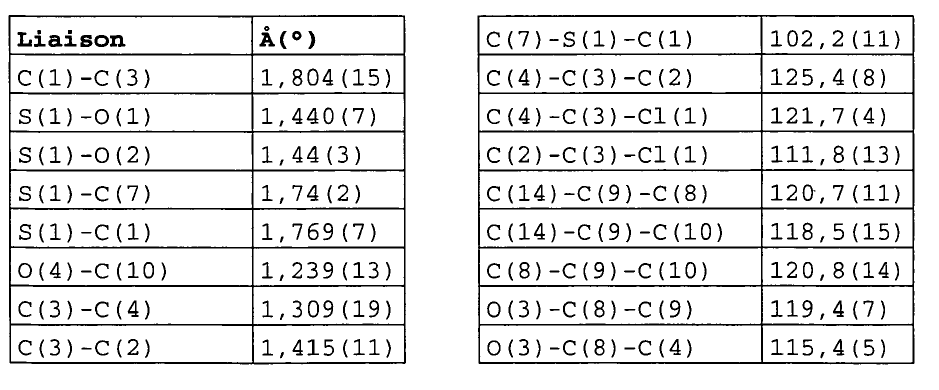

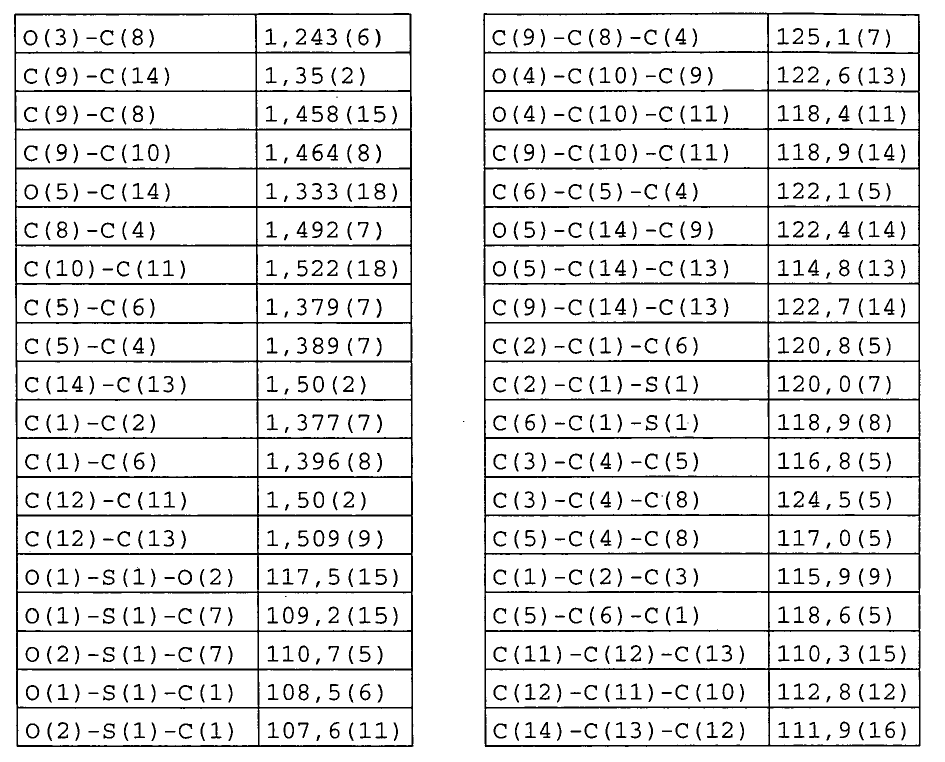

5. Modification cristalline thermodynamiquement stable de Sulcotrione selon l'une des revendications 1 à 4 avec une masse volumique solide de 1,536 g/ml.

6. Procédé de préparation de la modification cristalline thermodynamiquement stable de Sulcotrione selon la revendication 1, **caractérisé en ce que**
a) l'on met en suspension et/ou on dissout la modification cristalline 1 métastable de Sulcotrione dans l'eau et/ou des solvants organiques inertes et
b) on la traite à des températures de 0 °C à 80 °C jusqu'à la transformation quantitative en la modification cristalline thermodynamiquement stable.

7. Procédé selon la revendication 6, **caractérisé en ce qu'**on utilise comme solvant des alcools avec 1 à 10 atomes de carbone ou des cétones avec 3 à 10 atomes de carbone.

8. Agent herbicide, **caractérisé par** une teneur en modification cristalline thermodynamiquement stable de Sulcotrione selon une des revendications 1 à 5 et des diluants et/ou des adjuvants tensio-actifs courants.

9. Agent herbicide, comprenant la modification cristalline thermodynamiquement stable de Sulcotrione selon une des revendications 1 à 5 et la modification cristalline métastable de Sulcotrione, **caractérisé en ce que** le mélange contient moins de 20 % en poids de la modification cristalline métastable.

10. Utilisation de la modification cristalline thermodynamiquement stable de Sulcotrione selon une des revendications 1 à 5 ou d'un agent selon la revendication 8 ou 9 pour la lutte contre des plantes non souhaitées.

11. Procédé de lutte contre des plantes non souhaitées, **caractérisé en ce que** l'on laisse agir la modification cristalline thermodynamiquement stable de Sulcotrione selon l'une des revendications 1 à 5 ou un agent selon la revendication 8 ou 9 sur les plantes non souhaitées et/ou leur espace vital.
